(19) Europäisches Patentamt
European Patent Office
Office européen des brevets

(11) Veröffentlichungsnummer : **0 094 911**
**B1**

(12) **EUROPÄISCHE PATENTSCHRIFT**

(45) Veröffentlichungstag der Patentschrift : **10.09.86**

(51) Int. Cl.[4] : **C 09 B 57/00**, C 08 K 5/34, C 07 D487/04

(21) Anmeldenummer : **83810202.8**

(22) Anmeldetag : **11.05.83**

# (54) Herstellung von Pyrrolo-(3,4-c)-pyrrolen.

(30) Priorität : **17.05.82 CH 3054/82**
**15.09.82 CH 5468/82**

(43) Veröffentlichungstag der Anmeldung : **23.11.83 Patentblatt 83/47**

(45) Bekanntmachung des Hinweises auf die Patenterteilung : **10.09.86 Patentblatt 86/37**

(84) Benannte Vertragsstaaten :
**AT BE CH DE FR GB IT LI NL SE**

(56) Entgegenhaltungen :
**EP-A- 0 061 426**
**TETRAHEDRON LETTERS, Nr. 29, 1974, Seiten 2549-2552, Pergamon Press, Oxford, GB; D.G. FARNUM et al.: "Attempted reformatskii reaction of benzonitrile, 1,4-diketo-3,6-diphenylpyrrolo[3,4-C]pyrrole. A lactam analogue of pentalene"**

(73) Patentinhaber : **CIBA-GEIGY AG**
**Klybeckstrasse 141**
**CH-4002 Basel (CH)**

(72) Erfinder : **Rochat, Alain Claude, Dr.**
**Bruderholzallee 25**
**CH-4059 Basel (CH)**
Erfinder : **Cassar, Luigi, Dr.**
**Via Orfeo 25**
**Bologna (IT)**
Erfinder : **Iqbal, Abul, Dr.**
**Im Schaiengarten 1**
**CH-4107 Ettingen (CH)**

**EP 0 094 911 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von 1,4-Diketopyrrolo-[3,4-c]-pyrrolen, die wertvolle Pigmente darstellen. Tetrahedron Lett. *1974*, 2549-52, beschreibt ein Verfahren zur Herstellung von 1,4-Diketo-3,6-diphenylpyrrolo-[3,4-c]-pyrrol ausgehend von Benzonitril und Bromessigsäureäthylester in Gegenwart von aktiviertem Zink-Kupfer. Doch sind die bisher erzielten Ausbeuten ungenügend. Wählt man hingegen als Ausgangsmaterial einen Bernsteinsäureester und ein aromatisches Nitril, so erhält man unter bestimmten Reaktionsbedingungen die gewünschten Pyrrolo-[3,4-c]-pyrrol-Pigmente in wesentlich höheren Ausbeuten. Ausserdem erhält man nach dem vorliegenden Verfahren neue Pyrrolo-[3,4-c]-pyrrole, die nach dem vorbekannten Verfahren nicht oder sehr schwer erhältlich sind.

Die Erfindung betrifft somit ein Verfahren zur Herstellung von 1,4-Diketo-pyrrolo-[3,4-c]-pyrrolen der Formel

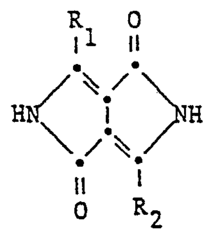

(I)

worin $R_1$ und $R_2$ unabhängig voneinander isocyclische oder heterocyclische aromatische Reste bedeuten, dadurch gekennzeichnet, dass man 1 Mol eines Bernsteinsäurediesters mit 2 Mol eines Nitrils der Formel

$R_1$—CN (II) oder $R_1$—CN (III)

oder mit 1 Mol eines Nitrils der Formel II und 1 Mol eines Nitrils der Formel III in einem organischen Lösungsmittel in Gegenwart einer starken Base bei erhöhter Temperatur umsetzt und aus dem entstandenen Reaktionsprodukt durch Hydrolyse die Verbindung der Formel I freisetzt. Die Reste $R_1$ und $R_2$ können verschieden oder gleich sein, vorzugsweise gleich. Bedeuten $R_1$ und $R_2$ isocyclische aromatische Reste, dann vorzugsweise mono- bis tetra-cyclische, insbesondere mono- oder bicyclische Reste, also Phenyl, Diphenylyl oder Naphthyl. Bedeuten $R_1$ und $R_2$ heterocyclische aromatische Reste, dann vorzugsweise mono- bis tricyclische. Diese können rein heterocyclisch sein oder einen heterocyclischen Ring und einen oder mehrere ankondensierte Benzolringe enthalten, wobei die Cyanogruppe jeweils sowohl am heterocyclischen wie am isocyclischen Teil gebunden sein kann. Beispiele von heterocyclischen aromatischen Resten sind Pyridyl, Pyrimidyl, Pyrazinyl, Triazinyl, Furoyl, Pyrrolyl, Thiophenyl, Chinolyl, Cumarinyl, Benzfuranyl, Benzimidazolyl, Benzoxazolyl, Dibenzfuranyl, Benzothiophenyl, Dibenzothiophenyl, Indolyl, Carbazolyl, Pyrazolyl, Imidazolyl, Oxazolyl, Isoxazolyl, Thiazolyl, Indazolyl, Benzthiazolyl, Pyridazinyl, Cinnolyl, Chinazolyl, Chinoxalyl, Phthalazinyl, Phthalazindionyl, Phthalamidyl, Chromonyl, Naphtholactamyl, Chinolonyl, ortho-Sulfobenzoesäureimidyl, Maleinimidyl, Naphtharidinyl, Benzimidazolonyl, Benzoxazolonyl, Benzthiazolonyl, Benzthiazothionyl, Chinazolonyl, Chinoxalonyl, Phthalazonyl, Dioxopyrimidinyl, Pyridonyl, Isochinolonyl, Isochinolinyl, Isothiazolyl, Benzisoxazolyl, Benzisothiazolyl, Indazolonyl, Acridinyl, Acridonyl, Chinazolindionyl, Chinoxalindionyl, Benzoxazindionyl, Benzoxazinonyl und Naphthalimidyl. Sowohl die isocyclischen wie die heterocyclischen aromatischen Reste können die üblichen nichtwasserlöslichmachenden Substituenten aufweisen, wie :

1) Halogenatome, beispielsweise Chlor, Brom oder Fluor.

2) Verzweigte oder unverzweigte Alkylgruppen mit vorzugsweise 1 bis 18, insbesondere 1 bis 12, vor allem 1 bis 8 und besonders bevorzugt mit 1 bis 4 C-Atomen. Diese Alkylgruppen können nichtwasserlöslichmachende Substituenten aufweisen, wie beispielsweise Fluor, Hydroxy, Cyano, —$OCOR_3$, —$OR_4$, —$COOR_3$, —$CONR_4R_5$ oder —$R_3$—$OCONHR_3$, worin $R_3$ Alkyl, Aryl, wie Naphthyl, oder unsubstituiertes oder durch Halogen, Alkyl oder —O-Alkyl substituiertes Benzyl oder einen heterocyclischen Rest, $R_4$ und $R_5$ Wasserstoff, unsubstituiertes oder durch Cyano oder Hydroxy substituiertes Alkyl, $C_5$-$C_6$-Cycloalkyl, Aryl oder Heteroaryl, insbesondere unsubstituiertes oder durch Halogen, Alkyl oder —O-Alkyl substituiertes Phenyl bedeuten, oder worin $R_4$ und $R_5$ zusammen mit dem N-Atom einen 5-6-gliedrigen Heteroring bilden, wie beispielsweise einen Morpholin-, Piperidin- oder Phthalimidring. Weitere mögliche Substituenten an den Alkylgruppen sind mono- oder dialkylierte Aminogruppen, Arylreste, wie Naphthyl oder insbesondere unsubstituiertes oder durch Halogen, Alkyl oder —O-Alkyl substituiertes Phenyl, oder ferner heterocyclische aromatische Reste, wie z. B. die 2-Thienyl-, 2-Benzoxazolyl-, 2-Benzthiazolyl-, 2-Benzimidazolyl-, 6-Benzimidazolonyl-, 2-, 3- oder 4-Pyridyl-, 2-, 4- oder 6-Chinolylreste.

Enthalten die unter 2) genannten Substituienten ihrerseits wieder Alkyl, so kann dieses Alkyl verzweigt oder unverzweigt sein und vorzugsweise 1 bis 18, insbesondere1 bis 12, vor allem 1 bis 8 und besonders bevorzugt 1 bis 4 C-Atomen enthalten.

Beispiele von unsubstituierten oder substituierten Alkylgruppen sind Methyl, Aethyl, n-Propyl, Isopropyl, n-Butyl, sec.-Butyl, tert.-Butyl, tert.-Amyl, n-Pentyl, n-Hexyl, 1,1,3,3-Tetramethylbutyl, n-Heptyl, n-Octyl, Nonyl, Decyl, Undecyl, Dodecyl, Hydroxymethyl, Trifluormethyl, Trifluoräthyl, Cyanmethyl, Methoxycarbonylmethyl, Acetoxymethyl oder Benzyl.

3) Die Gruppe $—OR_6$, worin $R_6$ Wasserstoff, Alkyl, Aryl, beispielsweise Naphthyl oder insbesondere unsubstituiertes oder durch Halogen, Alkyl, oder —O-Alkyl substituiertes Phenyl, $C_5$-$C_6$-Cycloalkyl, Aralkyl oder einen heterocyclischen Rest bedeutet. In den Definitionen von $R_6$ vorkommendes Alkyl kan z. B. eine der unter 2) als bevorzugt angegebenen Anzahl C-Atome haben. Als Beispiele von $R_6$ seien genannt : Methyl, Aethyl, n-Propyl, Isopropyl, Trifluoräthyl, Phenyl, o-, m- oder p-Chlorphenyl, o-, m- oder p-Methylphenyl, α-oder β-Naphthyl, Cyclohexyl, Benzyl, Thienyl oder Pyranylmethyl.

4) Die Gruppe $—SR_6$, worin $R_6$ die unter 3) angegebene Bedeutung hat. Als Beispiele für $R_6$ seien genannt : Methyl, Aethyl, n-Propyl, Isopropyl, Phenyl, o-, m- oder p-Chlorphenyl, o-, m- oder p-Methylphenyl, α- oder β-Naphthyl, Cyclohexyl, Benzyl, Thienyl oder Pyranylmethyl.

5) Die Cyangruppe.

6) Die Gruppe der Formel $—NR_4R_5$, worin $R_4$ und $R_5$ die unter 2) angegebene Bedeutung haben. Als Beispiele seien genannt : Amino, Methylamino, Dimethylamino, Aethylamino, Diäthylamino, Isopropylamino, β-Hydroxyäthylamino β-Hydroxypropylamino, N,N-Bis-(β-hydroxyäthyl)-amino, N,N-Bis-(β-cyanäthyl)-amino, Cyclohexylamino, Phenylamino, N-Methylphenylamino, Benzylamino, Dibenzylamino, Piperidyl oder Morpholyl.

7) Die Gruppe der Formel $—COOR_3$, worin $R_3$ die unter 2) angegebene Bedeutung hat. Als Beispiele für $R_3$ seien genannt : Methyl, Aethyl, Isopropyl, tert.-Butyl, n-Butyl, Phenyl, Benzyl oder Furfuryl.

8) Die Gruppe der Formel $—COR_6$, worin $R_6$ die unter 3) angegebene Bedeutung hat. Als Beispiele seien genannt : Methyl, Aethyl, tert.-Butyl, Phenyl, o-, m- oder p-Chlorphenyl, o-, m- oder p-Methylphenyl oder α- bzw. β-Naphthyl.

9) Die Gruppe der Formel $—NR_7COR_3$, worin $R_3$ die unter 2) angegebene Bedeutung hat, $R_7$ Wasserstoff, Alkyl, Aryl, beispielsweise Naphthyl oder insbesondere unsubstituiertes oder durch Halogen, Alkyl oder —O-Alkyl substituiertes Phenyl, $C_5$-$C_6$-Cycloalkyl, Aralkyl oder den Rest $—COR_3$ bedeutet, wobei zwei Reste $—COR_3$ zusammen mit dem N-Atom einen heterocyclischen Ring bilden können. In den Definitionen von $R_7$ vorkommendes Alkyl kann z. B. eine der unter 2) als bevorzugt angegebenen Anzahl C-Atome haben. Als Beispiele seien genannt : Acetylamino, Propionylamino, Butyrylamino, Benzoylamino, p-Chlorbenzoylamino, p-Methylbenzoylamino, N-Methylacetylamino, N-Methyl-benzoylamino, N-Succinimido oder N-Phthalimido.

10) Die Gruppe der Formel $—NR_6COOR_3$, worin $R_3$ und $R_6$ die unter 2) bzw. 3) angegebene Bedeutung haben. Als Beispiele seien die Gruppen $—NHCOOCH_3$, $NHCOOC_2H_5$ oder $NHCOOC_6H_5$ genannt.

11) Die Gruppe der Formel $—NR_6CONR_4R_5$, worin $R_6$, $R_5$ und $R_4$ die unter 3) und 2) angegebene Bedeutung haben. Als Beispiele seien genannt : Ureido, N-Methylureido, N-Phenylureido oder N,N-2',4'-Dimethylphenylureido.

12) Die Gruppe der Formel $—NHSO_2R_3$, worin $R_3$ die unter 2) angegebene Bedeutung hat. Als Beispiele seien genannt : Methansulfonylamino, Phenylsulfonylamino, P-Toluylsulfonylamino oder β-Naphthylsulfonylamino.

13) Die Gruppen der Formel $—SO_2R_3$ oder $—SOR_3$, worin $R_3$ die unter 2) angegebene Bedeutung hat. Als Beispiele seien genannt : Methylsulfonyl, Aethylsulfonyl, Phenylsulfonyl, 2-Naphthylsulfonyl, Phenylsulfoxidyl.

14) Die Gruppe der Formel $—SO_2OR_3$, worin $R_3$ die unter 2) angegebene Bedeutung hat. Als Beispiele für $R_3$ seien genannt : Methyl, Aethyl, Phenyl, o-, m- oder p-Chlorphenyl, o-, m- oder p-Methylphenyl, α- oder β-Naphthyl.

15) Die Gruppe der Formel $—CONR_4R_5$, worin $R_4$ und $R_5$ die unter 2) angegebene Bedeutung haben. Als Beispiele seien genannt : Carbamoyl, N-Methylcarbamoyl, N-Aethylcarbamoyl, N-Phenylcarbamoyl, N,N-Dimethyl-carbamoyl, N-Methyl-N-Phenylcarbamoyl, N-α-Naphthylcarbamoyl, oder N-piperidylcarbamoyl.

16) Die Gruppe der Formel $—SO_2NR_4R_5$, worin $R_4$ und $R_5$ die unter 2) angegebene Bedeutung haben. Als Beispiele seien genannt : Sulfamoyl, N-Methylsulfamoyl, N-Aethylsulfamoyl, N-Phenylsulfamoyl, N-Methyl-N-phenylsulfamoyl oder N-Morpholylsulfamoyl.

17) Die Gruppe der Formel $—N = N-R_8$, worin $R_8$ den Rest einer Kupplungskomponente oder einen gegebenenfalls durch Halogen, Alkyl oder —O-Alkyl subsituierten Phenylrest bedeutet. In den Definitionen von $R_8$ vorkommendes Alkyl kann z. B. eine der unter 2) als bevorzugt angegebenen Anzahl C-Atome haben. Als Beispiele für $R_8$ seien genannt : die Aceto-acetarylid-, Pyrazolyl-, Pyridonyl-, o- oder p-Hydroxyphenyl-, o-Hydroxynaphthyl-, p-Aminophenyl-, oder p-N,N-Dimethylaminophenylreste.

18) Die Gruppe der Formel $—OCOR_3$, worin $R_3$ die unter 2) angegebene Bedeutung hat. Als Beispiele für $R_3$ seien genannt : Methyl, Aethyl, Phenyl, o-, m- oder p-Chlorphenyl.

19) Die Gruppe der Formel $—OCONHR_3$, worin $R_3$ die unter 2) angegebene Bedeutung hat. Als

Beispiele für $R_3$ seien genannt: Methyl, Aethyl, Phenyl, o-, m-oder p-Chlorphenyl.

Vorzugsweise verwendet man zur erfindungsgemässen Herstellung von Verbindungen der Formel I als Ausgangsstoff ein einheitliches Nitril der Formel II oder III. Bevorzugt verwendet man auch Nitrile der Formeln II und/oder III, worin $R_1$ und $R_2$ unsubstituiertes oder nicht-wasserlöslichmachende Substituenten aufweisendes Phenyl oder Naphthyl bedeuten.

Vor allem verwendet man als Ausgangsstoffe Nitrile der Formel IV

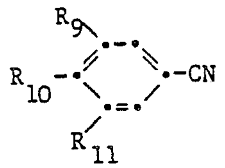

(IV)

worin $R_9$, $R_{10}$ und $R_{11}$ unabhängig voneinander Wasserstoff, Fluor, Chlor, Brom, Carbamoyl, Cyano, Trifluormethyl, $C_2$-$C_{13}$-Alkylcarbamoyl, $C_1$-$C_{12}$-Alkyl, $C_1$-$C_{12}$-Alkoxy, $C_1$-$C_{12}$-Alkylmercapto, $C_2$-$C_{13}$-Alkoxycarbonyl, $C_2$-$C_{13}$-Alkanoylamino, $C_1$-$C_{12}$-Monoalkylamino, $C_2$-$C_{24}$-Dialkylamino, unsubstituiertes oder durch Halogen, $C_1$-$C_{12}$-Alkyl oder $C_1$-$C_{12}$-Alkoxy substituiertes Phenoxy, Phenylmercapto, Phenoxycarbonyl, Phenylcarbamoyl oder Benzoylamino bedeuten, wobei mindestens einer der Substituenten $R_9$, $R_{10}$ und $R_{11}$ Wasserstoff bedeuten.

Insbesondere verwendet man als Ausgangsstoffe Nitrile der Formel V

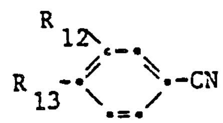

(V)

worin einer der Substituenten $R_{12}$ und $R_{13}$ Chlor, Brom, $C_1$-$C_4$-Alkyl, Cyano, $C_1$-$C_4$-Alkoxy, unsubstituiertes oder durch Chlor oder Methyl substituiertes Phenoxy, Carbamoyl, $C_2$-$C_5$-Alkylcarbamoyl, unsubstituiertes oder durch Chlor, Methyl oder Methoxy substituiertes Phenylcarbamoyl bedeutet, und der andere Wasserstoff ist.

Bei den zu verwendenden Bernsteinsäurediestern kann es sich um Dialkyl-, Diaryl- oder Monoalkylmonoarylester handeln, wobei auch die Bernsteinsäuredialkyl- und -diarylester unsymmetrisch sein können. Bevorzugt verwendet man aber symmetrische Bernsteinsäurediester, insbesondere symmetrische Bernsteinsäuredialkylester. Liegt ein Bernsteinsäurediaryl- oder -monoaryl-monoalkylester vor, so bedeutet Aryl insbesondere unsubstituiertes oder durch Halogen, wie Chlor, $C_1$-$C_6$-Alkyl, wie Methyl, Aethyl, Isopropyl oder tert.-Butyl, oder $C_1$-$C_6$-Alkoxy, wie Methoxy oder Aethoxy substituiertes Phenyl. Aryl bedeutet bevorzugt unsubstituiertes Phenyl. Handelt es sich um einen Bernsteinsäuredialkyl- oder -monoalkyl-monoarylester, so kann Alkyl unverzweigt oder verzweigt sein, bevorzugt verzweigt, und vorzugsweise 1 bis 18, insbesondere 1 bis 12, vor allem 1 bis 8 und besonders bevorzugt 1 bis 5 C-Atomen enthalten. Verzweigtes Alkyl ist bevorzugt sek.- oder tert.-Alkyl, wie z. B. Isopropyl, sek.-Butyl, tert.-Butyl, tert.-Amyl und Cyclohexyl.

Beispiele für Bernsteinsäurediester sind Bernsteinsäure-dimethylester, -diäthylester, -dipropylester, -dibutylester, -dipentylester, -dihexylester, -diheptylester, -dioctylester, -diisopropylester, -di-sec.-butylester, -di-tert.-butylester, -di-tert.-amylester, -di-[1,1-dimethylbutyl]-ester, -di-[1,1,3,3-tetramethylbutyl]-ester, -di-[1,1-dimethylpentyl]-ester, -di-[1-methyl-1-äthyl-butyl]-ester, -di-[1,1-diäthylpropyl]-ester, -diphenylester, -di-[4-methylphenyl]-ester, -di-[2-methylphenyl]-ester, -di-[4-chlorphenyl]-ester, -monoäthylmonophenylester, -dicyclohexylester.

Die Bernsteinsäure-diester und die Nitrile der Formel II bzw. III sind bekannte Verbindungen und können nach bekannten Verfahren hergestellt werden.

Man führt die Umsetzung des Bernsteinsäurediesters mit dem Nitril in einem organischen Lösungsmittel durch. Als Lösungsmittel eignen sich beispielsweise primäre, sekundäre oder tertiäre Alkohole mit 1 bis 10 C-Atomen, wie Methanol, Aethanol, n-Propanol, Isopropanol, n-Butanol, sek.-Butanol, tert.-Butanol, n-Pentanol, 2-Methyl-2-butanol, 2-Methyl-2-pentanol, 3-Methyl-3-pentanol, 2-Methyl-2-hexanol, 3-Aethyl-3-pentanol, 2,4,4-Trimethyl-2-pentanol oder Glykole, wie Aethylenglykol oder Diäthylenglykol, ferner Aether, wie Tetrahydrofuran oder Dioxan, oder Glykoläther, wie Aethylenglykol-methyläther, Aethylenglykol-äthyläther, Diäthylenglykol-monomethyläther oder Diäthylenglykol-monoäthyläther, ferner dipolar-aprotische Lösungsmittel, wie Acetonitril, Benzonitril, Dimethylformamid, N,N-Dimethylacetamid, Nitrobenzol, N-Methylpyrrolidon, aliphatische oder aromatische Kohlenwasserstoffe, wie Benzol oder durch Alkyl, Alkoxy oder Halogen substituiertes Benzol, wie Toluol, Xylol, Anisol oder Chlorbenzol oder aromatische N-Heterocyclen, wie Pyridin, Picolin oder Chinolin. Zudem ist es auch möglich, das umzusetzende Nitril der Formel II bzw. III gleichzeitig als Lösungsmittel zu verwenden, falls es im Temperaturbereich flüssig ist, in dem die Umsetzung erfolgt. Die genannten Lösungsmittel können

auch als Mischungen eingesetzt werden. Zweckmässigerweise verwendet man 5-20 Gew.-Teile Lösungsmittel auf 1 Gew.-Teil der Reaktionsteilnehmer.

Im erfindungsgemässen Verfahren verwendet man bevorzugt einen Alkohol als Lösungsmittel, insbesondere einen sekundären oder tertiären Alkohol. Bevorzugte tertiäre Alkohole sind tert.-Butanol und tert.-Amylalkohol. Dabei sind auch Gemische dieser bevorzugten Lösungsmittel mit aromatischen Kohlenwasserstoffen, wie Toluol oder Xylol, oder mit durch Halogen substituiertem Benzol, wie Chlorbenzol, von grossem Interesse.

Das erfindungsgemässe Verfahren wird in Gegenwart einer starken Base durchgeführt. Geeignete starke Basen sind insbesondere die Alkalimetalle selbst, wie Lithium, Natrium oder Kalium, oder Alkaliamide, wie Lithium-, Natrium- oder Kaliumamid oder Alkalihydride, wie Lithium-, Natrium- oder Kaliumhydrid, oder Erdalkali-oder Alkalialkoholate, die sich insbesondere von primären, sekundären oder tertiären aliphatischen Alkoholen mit 1 bis 10 C-Atomen ableiten, wie z. B. Lithium-, Natrium- oder Kaliummethylat, -äthylat, -n-propylat, -isopropylat, n-butylat, -sek.-butylat, tert.-butylat, -2-methyl-2-butylat, -2-methyl-2-pentylat, -3-methyl-3-pentylat, -3-äthyl-3-pentylat. Man kann aber auch ein Gemisch der genannten Basen verwenden.

Im erfindungsgemässen Verfahren verwendet man als starke Base bevorzugt Alkalialkoholate, wobei Alkali insbesondere Natrium oder Kalium bedeutet, und das Alkoholat sich bevorzugt von einem sekundären oder tertiären Alkohol ableitet. Besonders bevorzugte starke Basen sind daher z. B. Natrium- oder Kalium-isopropylat, -sek.-butylat, -tert.-butylat und -tert.-amylat. Dabei können die Alkalialkoholate auch *in situ* hergestellt werden, indem man den entsprechenden Alkohol mit dem Alkalimetall, Alkalihydrid oder Alkaliamid umsetzt.

Im erfindungsgemässen Verfahren kann man die starke Base in einer Menge von insbesondere 0,1 bis 10 Mol, bevorzugt 1,9 bis 4,0 Mol, bezogen auf 1 Mol des Reaktanden Bernsteinsäurediester einsetzen. Obwohl grundsätzlich stöchiometrische Mengen an Base genügen, beeinflusst in vielen Fällen ein Basenüberschuss die Ausbeute günstig.

Das erfindungsgemässe Verfahren wird insbesondere bei einer Temperatur von 60 bis 140 °C, vorzugsweise 80 bis 120 °C, durchgeführt.

Zur Hydrolyse des Kondensationsproduktes kann man Wasser, einen Alkohol mit 1 bis 4 C-Atomen, wie Methanol oder Aethanol, vorzugsweise aber eine Säure, verwenden. Als Säuren kommen z. B. aliphatische oder aromatische Carbon- oder Sulfonsäuren in Betracht, wie beispielsweise Ameisensäure, Essigsäure, Propionsäure, Oxalsäure, Benzoesäure oder Benzolsulfonsäure. Weiterhin kommen als Säuren auch Mineralsäuren in Betracht, wie Salzsäure, Schwefelsäure oder Phosphorsäure. Vorzugsweise benutzt man zur Hydrolyse eine organische Säure, insbesondere eine aliphatische Carbonsäure, wie Essigsäure.

Bei der Hydrolyse fällt die Verbindung der Formel I aus und kann durch Abfiltrieren isoliert werden.

Zur Umsetzung des Bernsteinsäurediesters mit den Nitrilen der Formel II bis V ist es grundsätzlich möglich, bei tieferer Temperatur alle Komponenten vorzulegen und dann das Gemisch in den Bereich der Reaktionstemperatur zu erwärmen, oder in beliebiger Reihenfolge die einzelnen Komponenten im Bereich der Reaktionstemperatur zueinander zuzugeben. Eine bevorzugte Ausführungsform, die sich in der Regel besonders günstig auf die Ausbeute auswirkt, besteht darin, dass man das umzusetzende Nitril zusammen mit der Base vorlegt und den Bernsteinsäurediester im Bereich der Reaktionstemperatur zudosiert. Eine weitere Möglichkeit besteht darin, dass man den Bernsteinsäurediester und das umzusetzende Nitril gleichzeitig zur vorgelegten Base zudosiert. Es ist durchaus möglich, das erfindungsgemässe Verfahren nicht nur batchweise, sondern auch kontinuierlich durchzuführen.

Insbesondere bei Bernsteinsäurediestern mit niederen Alkylresten und bei Alkoholaten, die sich aus niederen Alkoholen ableiten, wie z. B. Methanol, Aethanol, n-Propanol, Isopropanol oder tert.-Butanol, kann es sich als notwendig erweisen, den bei der Umsetzung entstehenden niederen Alkohol laufend aus dem Reaktionsmilieu zu entfernen, um höhere Ausbeuten zu erzielen.

Verwendet man als Lösungsmittel einen Alkohol und als Base ein Alkoholat, so kann es vorteilhaft sein, einen Alkohol und ein Alkoholat mit gleichen Alkylteilen zu wählen. Ebenso vorteilhaft kann es sein, wenn zudem noch der Bernsteinsäurediester ebensolche Alkylgruppen enthält.

Eine weitere bevorzugte Ausführungsform des Verfahrens besteht darin, das mit dem Bernsteinsäurediester umzusetzende Nitril in mehr als nur stöchiometrischen Mengen einzusetzen. Es hat sich nämlich gezeigt, dass bei einem Ueberschuss an Nitril bezüglich des Bernsteinsäurediesters, wobei das Optimum nach den jeweiligen Reaktionspartnern bestimmt werden muss und bis zu einem Zehnfachen der stöchiometrisch nötigen Menge bezüglich des Bernsteinsäurediesters betragen kann, die Ausbeute an Endprodukt in der Regel noch verbessert werden kann. Das überschüssige Nitril lässt sich in der Regel zurückgewinnen. In manchen Fällen kann sich ein Ueberschuss an Bernsteinsäurediester bezüglich des Nitrils positiv auf die Ausbeute auswirken, wobei der Ueberschuss bis zur doppelten Menge des stöchiometrisch benötigten Bernsteinsäurediesters betragen kann.

Die nach dem erfindungsgemässen Verfahren hergestellten Verbindungen der Formel I sind neu, soweit $R_1$ und $R_2$ Reste aromatischer N-Heterocyclen bedeuten. Diese N-Heterocyclen können unsubstituiert oder durch Halogen, Cyano, Carbamoyl, Trifluormethyl, Alkyl oder Alkoxy substituiert sein, wobei Alkyl oder Alkoxy bevorzugt 1 bis 18, besonders 1 bis 8 und vor allem 1 bis 4 C-Atome aufweisen. $R_1$ und $R_2$ können beispielsweise Reste von Pyrrol, Indol, Pyrazol, Imidazol, Benzimidazol, Oxazol, Isoxazol,

Benzoxazol, Thiazol, Isothiazol, Benzisothiazol, Indazol, Pyridin, Chinolin, Isochinolin, Pyridazin, Pyrazin, Pyrimidin, 1,2,4- und 1,3,5-Triazin, Acridin, Cinnolin, Chinazolin, Chinoxalin oder Naphtharidin sein. Als Reste von aromatischen N-Heterocyclen bedeuten $R_1$ und $R_2$ bevorzugt Chinolyl, Isochinolyl, besonders bevorzugt aber o-, m- oder p-Pyridyl.

Je nach Art ihrer Substituenten und der zu färbenden Polymeren können die Verbindungen der Formel I auch als polymerlösliche Farbstoffe verwendet werden. In der Regel jedoch verwendet man die Verbindungen der Formel I als Pigmente für hochmolekulare organische Materialien. Dabei lassen sich die Pigmente im allgemeinen direkt in der Form einsetzen, wie sie nach dem erfindungsgemässen Verfahren anfallen.

Je nach Verwendungszweck kann man die nach dem erfindungsgemässen Verfahren anfallenden Pigmente in eine deckendere oder transparentere Form überführen.

Um eine transparente Form zu erzielen, wird die Hydrolyse bevorzugt bei tieferen Temperaturen (unter 80 °C) durchgeführt.

Wird eine deckendere Pigmentform gewünscht, so erweist sich eine Hydrolyse bei höherer Temperatur (über 80 °C), gegebenenfalls unter Druck, als zweckmässig. Man kann auch das Pigment nach der Hydrolyse zuerst isolieren und nachträglich in Wasser oder in einem organischen Lösungsmittel erwärmen, gegebenenfalls unter Druck, um die deckende Form zu erlangen. Vorzugsweise verwendet man solche organische Lösungsmittel, die über 80 °C sieden. Als besonders geeignet erweisen sich durch Halogenatome, Alkyl- oder Nitrogruppen substituierte Benzole, wie Xylole, Chlorbenzol, o-Dichlorbenzol oder Nitrobenzol, sowie Pyridinbasen, wie Pyridin, Picolin oder Chinolin, ferner Ketone, wie Cyclohexanon, Aether, wie Aethylenglykolmonomethyl- oder -monoäthyläther, Amide, wie Dimethylformamid oder N-Methylpyrrolidon, sowie Dimethylsulfoxid oder Sulfolan. Man kann die Nachbehandlung auch in Wasser in Gegenwart von organischen Lösungsmitteln und/oder mit Zusatz von oberflächenaktiven Substanzen durchführen.

Je nach Verwendungszweck kann es vorteilhaft sein, Gemische von Verbindungen der Formel I herzustellen. Dies lässt sich beispielsweise dadurch erreichen, dass man unabhängig voneinander hergestellte, verschiedene Reaktionslösungen vor der Hydrolyse mischt, zusammen hydrolysiert und dann isoliert. Es ist auch möglich zwei oder mehr Verbindungen der Formel I zusammen umzufällen.

Hochmolekulare organische Materialien, die mit den Verbindungen der Formel I gefärbt bzw. pigmentiert werden können, sind z. B. Cellulose-äther und -ester, wie Aethylcellulose, Nitrocellulose, Celluloseacetat, Cellulosebutylat, natürliche Harze oder Kunstharze, wie Polymerisationsharze oder Kondensationsharze, z. B. Aminoplaste, insbesondere Harnstoff- und Melamin-Formaldehydharze, Alkydharze, Phenoplaste, Polycarbonate, Polyolefine, wie Polystyrol, Polyvinylchlorid, Polyäthylen, Polypropylen, Polyacrylnitril, Polyacrylsäureester, Polyamide, Polyurethane oder Polyester, Gummi, Casein, Silikon und Silikonharze, einzeln oder in Mischungen.

Dabei spielt es keine Rolle, ob die erwähnten hochmolekularen organischen Verbindungen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemäss zu verwendenden Pigmente als Toner oder in Form von Präparaten einzusetzen. Bezogen auf das zu pigmentierende hochmolekulare organische Material setzt man die Verbindungen der Formel I in einer Menge von vorzugsweise 0,1 bis 10 Gew.% ein.

Die erhaltenen Färbungen, beispielsweise in Kunststoffen, Fasern, Lacken oder Drucken, zeichnen sich durch grosse Farbstärke, gute Dispergierbarkeit, gute Ueberlackier-, Migrations-, Hitze-, Licht- und Wetterechtheit, sowie durch einen guten Glanz aus.

Die folgenden Beispiele erläutern die Erfindung.

## Beispiel 1

Ein möglichst wasserfreies Gemisch aus 48,2 ml tert.-Amylalkohol, 17,3 g Kalium-tert.-butylat und 72,2 g Benzonitril wird unter Stickstoff-Atmosphäre auf ca. 98 °C erwärmt. Sobald diese Temperatur erreicht ist, wird eine möglichst wasserfreie Lösung von 7,31 g Bernsteinsäure-dimethylester in 5,0 ml tert.-Amylalkohol innert 145 Minuten mit einer Dosierpumpe zugefügft. Die Temperatur wird stets bei 98-99 °C gehalten. Das entstehende Methanol destilliert ab. Am Ende der Zudosierung wird das Reaktionsgemisch noch 2 Stunden bei 99 °C gehalten. Man kühlt anschliessend auf 65 °C, verdünnt langsam mit 100 ml Methanol, neutralisiert langsam mit 10,8 ml Eisessig und kocht kurz bei Rückflusstemperatur. Die erhaltene Pigment-Suspension wird bei ca. 50 °C filtriert. Der Filterkuchen wird in 300 ml Methanol aufgeschlämmt, und das Pigment wird wieder filtriert. Schliesslich wäscht man es farblos mit Methanol und Wasser und trocknet es bei 80 °C im Vakuum. Man erhält 9,04 g (entsprechend 62,8 % d. Th. bezogen auf Bernsteinsäure-dimethylester) reines Pigment der Formel VI

(Siehe Figur Seite 7 f.)

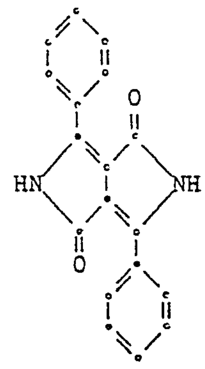

(VI)

das in PVC eingearbeitet eine rote Färbung ergibt.

Anstelle von Bernsteinsäure-dimethylester kann ohne besonderen Nachteil Bernsteinsäure-diäthylester verwendet werden.

Beispiel 2

23 g Kalium-t-butylat werden in 100 ml trockenem tert.-Amylalkohol aufgeschlämmt und weitgehend gelöst. Man fügt 20,6 g Benzonitril zu und heizt das Gemisch auf ca. 97 °C. Sobald diese Temperatur erreicht ist, lässt man unter Rühren eine Lösung aus 23,0 g Bernsteinsäure-di-tert.-butylester und 10 ml tert.-Amylalkohol innert 3 1/4 Stunden mittels Dosierpumpe einlaufen. Man hält die Reaktionstemperatur bei 96-98 °C und destilliert teilweise den entstehenden tert.-Butylalkohol ab. Nach beendeter Zudosierung wird das Reaktionsgemisch noch 2 Stunden bei 95-97 °C gehalten und dann wie in Beispiel 1 aufgearbeitet, wobei man anstelle von 10,8 ml 13,2 ml Eisessig verwendet. Nach dem Trocknen bei 80 °C im Vakuum erhält man 17,6 g reines Pigment der Formel VI, entsprechend 60,9 % d. Th. bezogen auf den eingesetzten Ester. Aus der Mutterlauge kann nicht umgesetztes Benzonitril zurückgewonnen werden.

Beispiel 3

4,6 g Natrium zuerst in 65 ml sek.-Butylalkohol bei Rückflusstemperatur (ca. 97 °C) gelöst (etwa 5 Stunden). Man kühlt die erhaltene Lösung auf ca. 50 °C, gibt 51,6 g Benzonitril hinzu und erwärmt das Gemisch auf 97 °C. Anschliessend lässt man 23,0 g Bernsteinsäure-di-sek.-butylester innert ca. 3 Stunden mittels einer Dosierpumpe einlaufen und hält stets die Umsetzungstemperatur bei 97 °C (Rückflusstemperatur). Nach beendeter Zudosierung wird das Gemisch noch 1 1/2 Stunden bei 97 °C gehalten und dann wie in Beispiel 1 aufgearbeitet (man verwendet 12,6 ml Eisessig anstelle von 10,8 ml). Nach dem Trocknen bei 80 °C im Vakuum erhält man 8,7 g reines Pigment der Formel VI (entsprechend 30,2 % d. Th. bezogen auf den eingesetzten Ester).

Beispiele 4-14

23,0 g Kalium-tert.-butylat werden in ca. 95 ml trockenen tert.-Amylalkohol suspendiert und durch Rühren weitgehend gelöst. 0,2 Mol des Nitrils der Formel R-CN, wobei R die in Tabelle I angegebene Bedeutung hat, werden anschliessend zugegeben. Man erwärmt das Gemisch auf die in Tabelle I angegebene Temperatur. Sobald diese Reaktionstemperatur erreicht ist, lässt man eine Lösung, bestehend aus 13,25 ml Bernsteinsäure-dimethylester und 5 ml tert.-Amylalkohol innerhalb der in Tabelle I angegebenen Zeit mittels Dosierpumpe einlaufen. Dabei wird leicht gerührt. Man hält die angegebene Temperatur und destilliert das entstehende Methanol ab. Wenn das Gemisch zu dickflüssig wird, kann es mit etwas tert.-Amylalkohol verdünnt werden. Nach beendeter Zudosierung wird das Reaktionsgemisch noch 2 Stunden bei derselben Temperatur gehalten und wie in Beispiel 1 aufgearbeitet, wobei man 13,2 ml Eisessig anstelle von 10,8 ml verwendet. Nach dem Trocknen im Vakuum bei 80 °C erhält man in der in Tabelle I angegebenen Ausbeute das Pigment der Formel

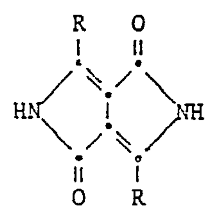

worin R die in Tabelle I angegebene Bedeutung hat.

Tabelle I

| Bsp. Nr. | R | Reaktionstemperatur in °C | Einlaufzeit in Stunden | Ausbeute in % der Theorie bezogen auf Bernsteinsäure-diester | Nuance in PVC (0,2 %) |
|---|---|---|---|---|---|
| 4 | 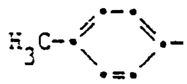 | 97-99 | 3 3/4 | 23,4 | rot |
| 5 | 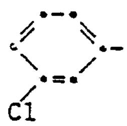 | 89-91 | 2 | 56,8 | orange |
| 6 | 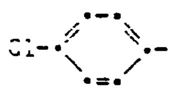 | 88-91 | 2 | 39,5 | bordeaux |
| 7 | 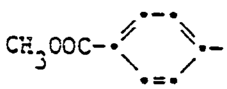 | 89-91 | 2 1/4 | 6,6 | rot |
| 8 | 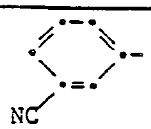 | 89-91 | 2 1/4 | 77,5 | rot-orange |
| 9 | 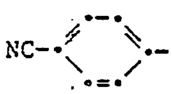 | 90-91 | 2 1/2 | 80,0 | bordeaux |
| 10 | 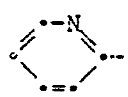 | 89-90 | 1 3/4 | 43,1 | rot |
| 11 | 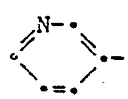 | 89-91 | 1 3/4 | 67,6 | rot |
| 12 | 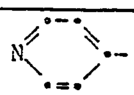 | 87-92 | 2 | 76,5 | rot |
| 13 | 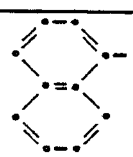 | 95-97 | 2 | 4,5 | orange |
| 14 | 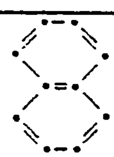 | 96-97 | 1 3/4 | 24,2 | rot |

Beispiele 15-37

Zu 145 ml tert.-Amylalkohol werden unter Stickstoffbegasung 8,3 g Natrium und 0,12 g Sulfobernsteinsäure-bis-2-acetyl-hexylester-Natriumsalz zugegeben. Das Gemisch wird unter leichtem Rühren bei 95-102 °C erwärmt. Sobald das Natrium geschmolzen ist, wird die Emulsion während 3 bis 5 Stunden bei 95-102 °C kräftig gerührt. Die so erhaltene Lösung versetzt man einerseits mit 0,24 Mol des Nitrils der Formel R'-CN oder R"-CN, wobei R' und R" gleich sind und die in Tabelle II angegebene Bedeutung haben (Beispiele 15 bis 25), oder andererseits mit 0,12 Mol des Nitrils der Formel R'-CN und 0,12 Mol des Nitrils der Formel R"-CN, wobei R' und R" verschieden sind und die in Tabelle III angegebene Bedeutung haben (Beispiele 26-37). Mittels einer Dosierpumpe gibt man bei der in Tabelle II und III angegebenen Reaktionstemperatur innerhalb der in Tabelle II und III angegeben Einlaufszeit 1,2 Mol in 12 ml tert.-Amylalkohol gelösten Bernsteinsäurediisopropylester. Das entstehende Isopropanol wird laufend abdestilliert. Nach beendeter Zudosierung wird das Gemisch noch 2 Stunden bei der Reaktionstemperatur gehalten und dann analog zu Beispiel 1 aufgearbeitet. Nach dem Trocknen im Vakuum bei 80 °C erhält man in der Tabelle II und III angegebenen Ausbeute das Pigment der Formel

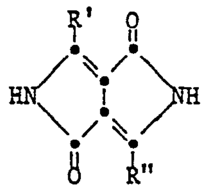

worin R' und R" die in Tabelle II bzw. III angegebene Bedeutung haben.

Tabelle II

| Bsp. Nr. | R'=R" | Reaktionstemperatur in °C | Einlaufzeit in Stunden | Ausbeute in % der Theorie bezogen auf Bernsteinsäure-diester | Nuance in PVC (0,2 %) |
|---|---|---|---|---|---|
| 15 | 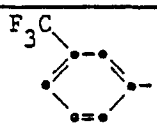 | 105-110 | 3 | 56,8 | orange-gelb |
| 16 | 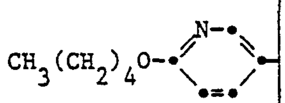 | 105-110 | 3 | 65,0 | rot |
| 17 | 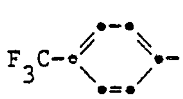 | 105-110 | 2,5 | 44,9 | rot |
| 18 | 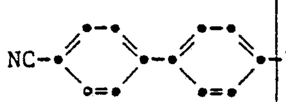 | 105-110 | 3 | 36,7 | rot-violett |
| 19 | 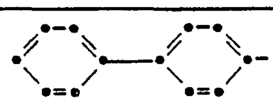 | 105-110 | 2,5 | 10,0 | bordeaux |
| 20 | 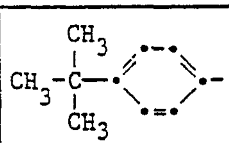 | 105-110 | 2 | 55,2 | rot |

Tabelle II (Fortsetzung)

| Bsp. Nr. | R'=R'' | Reaktionstemperatur in °C | Einlaufzeit in Stunden | Ausbeute in % der Theorie bezogen auf Bernsteinsäure-diester | Nuance in PVC (0,2 %) |
|---|---|---|---|---|---|
| 21 | 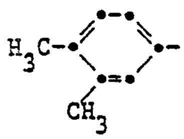 | 105-110 | 2 | 52,4 | rot |
| 22 | 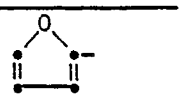 | 90 | 1 | 17,9 | violett |
| 23 | 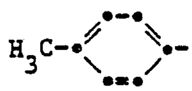 | 105-110 | 2 | 41,8 | rot |
| 24 | 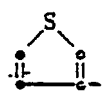 | 85 | 1 | 42,0 | bordeaux |
| 25 | 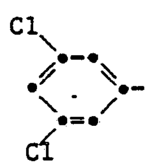 | 85 | 1 | 70,4 | rot |

Tabelle III

| Bsp. Nr. | R' | R'' | Reaktionstemperatur in °C | Einlaufzeit in Stunden | Ausbeute in % der Theorie bezogen auf Bernsteinsäure-diester | Nuance in PVC (0,2 %) |
|---|---|---|---|---|---|---|
| 26 | 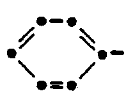 | 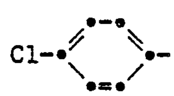 | 105-110 | 2,5 | 66,9 | rot |
| 27 | 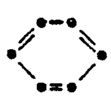 | 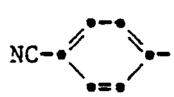 | 90 | 1,3 | 64,1 | rot-violett |
| 28 | 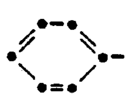 | 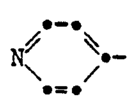 | 105-110 | 2,5 | 52,2 | rot |

Tabelle III (Fortsetzung)

| Bsp. Nr. | R' | R" | Reaktionstemperatur in °C | Einlaufzeit in Stunden | Ausbeute in % der Theorie bezogen auf Bernsteinsäure-diester | Nuance in PVC (0,2%) |
|---|---|---|---|---|---|---|
| 29 | 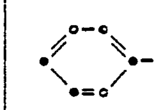 | 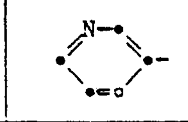 | 105-110 | 2,5 | 49,5 | orange |
| 30 | 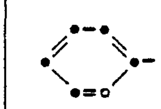 | 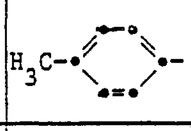 | 105-110 | 2,5 | 54,6 | rot |
| 31 | 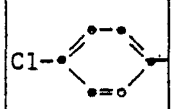 | 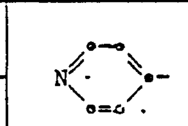 | 105-110 | 2,5 | 71,4 | rot |
| 32 | 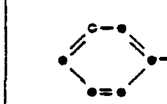 | 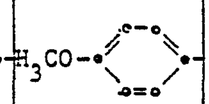 | 105-110 | 1 | 47,8 | rot |
| 33 | 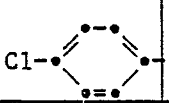 | 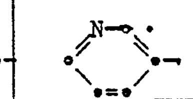 | 105-110 | 2,5 | 44,5 | rot |
| 34 | 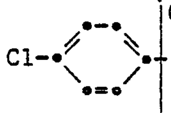 | 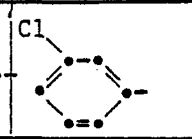 | 104 | 2,5 | 68,3 | orange |
| 35 | 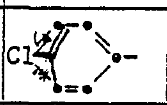 | 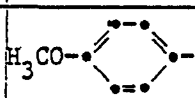 | 105-110 | 2,5 | 56,1 | bordeaux |
| 36 | 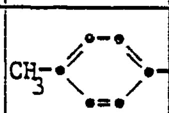 | 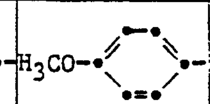 | 105-110 | 2,5 | 46,3 | rot |
| 37 | 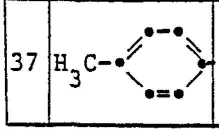 | 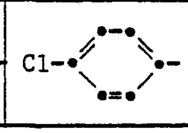 | 105-110 | 2,5 | 56,4 | bordeaux |

Beispiel 38

4,6 g Natrium und 0,1 g Natrium-laurylsulfat als Emulgator werden in 117 ml tert.-Amylalkohol so lange bei 94 bis 100 °C kräftig gerührt, bis das Natrium vollständig gelöst ist. Nach dem Abkühlen fügt man 25,6 g trockenes Isophthalsäuredinitril hinzu und lässt bei einer Temperatur von 88 bis 92 °C innerhalb von 2 Stunden eine Lösung von 13,25 ml Bernsteinsäure-dimethylester und 5 ml tert.-Amylalkohol mittels einer Dosierpumpe zulaufen. Man hält unter Rühren die Temperatur bei 88-92 °C und destilliert laufend das entstehende Methanol ab. Nach beendeter Zudosierung wird das Reaktionsgemisch noch 2 Stunden bei 90 °C gehalten und anschliessend wie in Beispiel 1 aufgearbeitet, wobei man 13,2 ml anstellen von 10,8 ml Eisessig verwendet. Nach dem Trocknen im Vakuum bei 80 °C erhält man 25,5 g (75,5 % d. Th. bezogen auf Bernsteinsäure-dimethylester) des Pigmentes der Formel

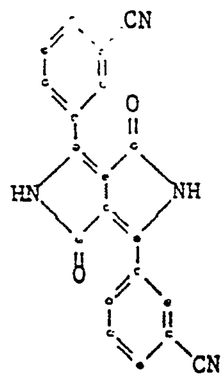

(VII)

das in PVC eingearbeitet eine rot-orange Färbung ergibt.

Beispiel 39

Geht man wie in Beispiel 38 vor, setzt aber überall statt tert.-Amylalkohol sek.-Butylalkohol ein, so erhält man 20,6 g (60,8 % d. Th. bezogen auf den Ester) des Pigmentes der Formel VII.

Beispiel 40

Geht man wie in Beispiel 38 vor, verwendet als Lösungsmittel tert.-Amylalkohol und als Alkoholat *in situ* hergestelltes Natrium-tert.-butylat, so erhält man 22,9 g (67,8 % d. Th. bezogen auf den Ester) des Pigmentes der Formel VII.

Beispiel 41

11,5 g Kalium-tert.-butylat werden in 17,4 ml tert.-Amylalkohol und 102,6 ml Benzonitril vorgelegt. Das Gemisch wird auf 100 °C erwärmt und bei dieser Temperatur mit einer Lösung von 11,6 g Bernsteinsäure-di-tert.-butylester in 5 ml tert.-Amylalkohol innerhalb 3 Stunden versetzt (Dosierpumpe). Nach 2 Stunden bei 100 °C, wird das Reaktionsgemisch wie in Beispiel 1 aufgearbeitet, wobei man anstelle von 10,8 ml 7,2 ml Eisessig verwendet. Nach dem Trocknen bei 80 °C im Vakuum erhält man 10,15 g (70,4 % d. Th. bezogen auf den Ester) reines Pigment der Formel VI.

Beispiel 42

11,5 g Kalium-tert.-butylat werden in 53,2 ml tert.-Amylalkohol suspendiert und mit 36,8 ml Benzonitril versetzt. Das Gemisch wird auf 98 °C erwärmt und bei dieser Temperatur mit einer Lösung von 13,6 g Bernsteinsäure-diphenylester in 35 ml Benzonitril innert 2 3/4 Stunden versetzt. Nach 1 1/2 Stunden bei 100 °C wird das Reaktionsgemisch wie in Beispiel 1 aufgearbeitet, wobei man 6,9 ml Eisessig anstelle von 10,8 verwendet. Nach dem Trocknen in Vakuum bei 80 °C erhält man 1,66 g (11,5 % d. Th. bezogen auf den Ester) des Pigmentes der Formel VI.

Beispiel 43

11,5 g Kalium-tert.-butylat werden in 100 ml Toluol suspendiert, und das Gemisch wird auf 90 °C erwärmt. Innert 2 Stunden wird anschliessend eine Lösung von 6,63 ml Bernsteinsäure-dimethyl-ester und 25,6 ml Benzonitril zudosiert. Das Reaktionsgemisch wird noch 16 Stunden bei 90 °C gerührt und dann wie in Beispiel 1 aufgearbeitet, wobei man 7,2 ml Eissessig anstelle von 10,8 verwendet. Nach dem Trocknen bei 80 °C im Vakuum erhält man 2,54 g (17,6 % d. Th. bezogen auf den Ester) reines Pigment der Formel VI.

Beispiel 44

4,6 g Natrium und 0,1 g Natriumlaurylsulfat werden in 70 ml tert.-Amylalkohol vorgelegt. Man erwärmt diese Suspension auf Rückflusstemperatur (95-100 °C). Innert 2 Stunden werden dann 20,7 ml tert.-Butylalkohol zugetropft. Das Gemisch wird solange bei Rückflusstemperatur (95-100 °C) gekocht, bis das Metall vollständig gelöst ist.

Nach Abkühlen auf Raumtemperatur gibt man 59,2 g 4-Tolunitril zu und erwärmt auf 97 °C. Anschliessend dosiert man eine Lösung von 22,1 g Bernsteinsäure-monoisopropyl-mono-tert.-butylester in 10,0 ml tert.-Amylalkohol innert ca. 4 Stunden zu und hält stets die Temperatur bei 97-99 °C. Anschliessend rührt man noch 1 1/2 Stunden bei derselben Temperatur und arbeitet wie in Beispiel 1 auf, wobei man 13,2 ml Eisessig anstelle von 10,8 ml verwendet. Nach dem Trocknen bei 80 °C im Vakuum

erhält man 15,4 g (48,6 % d. Th. bezogen auf den Ester) des Pigmentes der Formel VIII, das in PVC eingearbeitet eine rote Färbung ergibt.

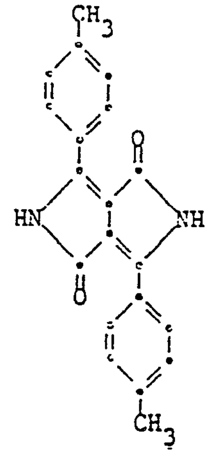

(VIII)

Beispiel 45

4,6 g Kalium-tert.-butylat werden in 20 ml 3-Methyl-3-pentanol suspendiert und mit 5,94 g 4-Dimethylamino-benzonitril versetzt. Man erwärmt auf 120 °C und tropft innerhalb 2 Stunden eine Lösung von 2,65 ml Bernsteinsäuredimethylester in 6 ml 3-Methyl-3-pentanol zu. Nach 2 Stunden bei 120 °C wird wie in Beispiel 1 aufgearbeitet, wobei man 2,3 ml Eisessig anstelle von 10,8 ml verwendet. Nach dem Trocknen bei 80 °C im Vakuum erhält man 0,28 g (3,7 % d. Th. bezogen auf den Ester) des Pigmentes der Formel IX, das in PVC eingearbeitet eine blaue Färbung ergibt.

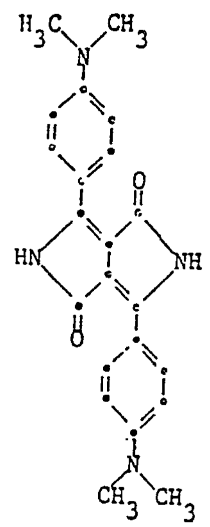

(IX)

Beispiel 46

23,0 g Kalium-tert.-butylat werden in 145 ml wasserfreiem tert.-Amylalkohol suspendiert und durch Rühren weitgehend gelöst. 12,8 g Terephthalonitril, sowie 12,8 g Isophthalonitril werden anschliessend zugegeben. Man erwärmt das Gemisch auf ca. 90 °C und gibt dann eine Lösung aus 13,25 ml Bernsteinsäure-dimethylester und 5 ml tert.-Amylalkohol innerhalb 2 1/2 Stunden mittels Dosierpumpe zu. Man hält die Temperatur bei ca. 90 °C und destilliert das entstehende Methanol ab. Nach beendeter Zugabe wird das Reaktionsgemisch noch 1/2 Stunden bei ca. 90 °C gehalten und wie in Beispiel 1 aufgearbeitet, wobei man 12,6 ml Eisessig anstelle von 10,8 ml verwendet. Nach dem Trocknen im Vakuum bei 80 °C erhält man 24,5 g (72,5 % d. Th. bezogen auf den Ester) des Pigmentes der Formel X, das in PVC eingearbeitet eine rote Färbung ergibt.

(Siehe Figur Seite 14 f.)

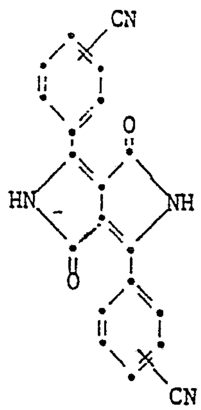

(X)

## Beispiel 47

3,4 g des Pigmentes von Beispiel 9 und 2,9 g des Pigmentes von Beispiel 12 in 100 ml wasserfreiem Dimethylformamid werden mit 7,43 ml einer 30 gew.-%-igen Lösung von Natriummethylat in Methanol versetzt. Nach etwa 3/4 Stunden Rühren bei Raumtemperatur tropft man innert 30 Minuten eine Lösung von 2,52 ml Eisessig in 60 ml Methanol zu und rührt die Suspension mehrere Stunden bei Raumtemperatur. Nach dem Filtrieren, Waschen und Trocknen erhält man 5,4 g eines Gemisches der Pigmente gemäss Beispiel 9 und 12.

## Beispiel 48

Zu 1 440 ml trockenem tert.-Amylalkohol werden unter Stickstoffbegasung 82,8 g Natrium und 1,2 g trockenes Sulfobernsteinsäure-bis-2-äthylhexylester-Natriumsalz als Emulgator gegeben. Das Gemisch wird auf ca. 100 °C erwärmt und bei Rückflusstemperatur gehalten, bis sich das Metall ganz aufgelöst hat. Die erhaltene Lösung wird auf ca. 80 °C abgekühlt und mit 247,2 g trockenem Benzonitril versetzt. Darauf erwärmt man das Gemisch auf ca. 110 °C und dosiert innerhalb ca. 6 Stunden 242,4 g trockenen Bernsteinsäurediisopropylester zu. Gleichzeitig destilliert man das entstehende Isopropanol ab. Nach beendeter Zudosierung lässt man 2 Stunden ausreagieren, kühlt das Gemisch auf ca. 60 °C ab und verdünnt es mit 1 650 ml Methanol. Dann gibt man langsam eine Mischung aus 227 ml Eisessig und 150 ml Methanol zu, wobei das Pigment ausfällt. Das Produkt wird bei ca. 60 °C filtriert und nacheinander mit 3 000 ml Methanol und 2 000 ml heissem Wasser gewaschen. Nach dem Trocknen im Vakuum bei 70 °C erhält man 228,3 g (66 % d. Th. bezogen auf Benzonitril) des Pigmentes der Formel VI.

## Beispiel 49

Verfährt man genau wie in Beispiel 48 angegeben, verwendet aber statt Bernsteinsäure-diisopropylester

a) Bernsteinsäure-dineopentylester,
b) Bernsteinsäure-di-2-butylester,
c) Bernsteinsäure-dicyclohexylester oder
d) Bernsteinsäure-di-tert.-butylester

so erhält man das Pigment der Formel VI in folgenden Ausbeuten (% der Theorie bezogen auf Benzonitril) :

Mit Ester a) : 64,9 %
Mit Ester b) : 65,2 %
Mit Ester c) : 71,5 %
Mit Ester d) : 75,7 %.

## Beispiel 50

Verfährt man genau wie in Beispiel 48 angegeben, erhöht aber die eingesetzten Natrium- und Bernsteinsäure-diisopropyl-ester-Mengen beide um 30 Gew.-%, so erhält man das Pigment der Formel VI in einer Ausbeute von 81,9 % der Theorie bezogen auf Benzonitril.

## Beispiel 51

6,9 g Natrium werden unter Argon in 100 ml Toluol und 26,5 g 2-Methyl-2-butanol vorgelegt. Das

Gemisch wird solange bei Rückflusstemperatur gerührt (ca. 100 °C), bis das Metall vollständig gelöst ist. Bei 70°-80 °C wird innert 4-5 Stunden ein Gemisch aus 21 ml Benzonitril, 23 g Bernsteinsäure-di-tert.-butylester und 20 ml Toluol zugetropft. Anschliessend wird die Suspension ca. 19 Stunden bei 80°-90 °C gerührt, dann bei 60 °C durch Zutropfen einer Mischung von 21 ml Eisessig und 80 ml Methanol innert ca. 1,5 Stunden neutralisiert und weitere 30 Minuten gerührt. Die Pigmentsuspension wird bei 60 °C filtriert, zuerst mit Methanol und dann mit Wasser farblos gewaschen. Nach dem Trocknen im Vakuumschrank bei 80 °C erhält man 23,8 g reines Pigment (= 82,5 % der Theorie bezogen auf Bernsteinsäure-di-tert.-butylester) des Beispiels 1 der Formel VI.

Beispiel 52

Unter Argon werden 3,4 g 45 gew.-%ige Natrium-Dispersion in Paraffin in 30 ml Toluol vorgelegt. Innert ca. 2 Stunden wird eine Mischung von 14 ml Benzonitril, 7,6 g Bernsteinsäure-di-tert.-butylester und 27 ml Toluol zugetropft. Die Temperatur wird von 20 °C auf 70 °C allmählich erhöht. Danach rührt man noch während ca. 20 Stunden bei 80 °C, wobei das Pigment ausfällt. Nach Neutralisation mit einer Mischung von 3,6 ml Eisessig und 27 ml Methanol wird die Suspension filtriert, mit Aceton und dann mit Wasser farblos gewaschen. Nach dem Trocknen im Vakuumschrank bei 80 °C erhält man 5,6 g Pigment (= 58,9 % d. Th. bezogen auf Bernsteinsäure-di-tert.-butylester) der Formel VI.

Beispiel 53

Geht man wie im Beispiel 48 vor, neutralisiert aber am Schluss mit 8 gew.-%iger Salzsäure, anstatt mit Eisessig/Methanol, so erhält man nach dem Trocknen 230 g reines Pigment (= 66,5 % d. Th. bezogen auf Bernsteinsäure-di-isopropylester) der Formel VI.

**Patentansprüche**

1. Verfahren zur Herstellung von 1,4-Diketo-pyrrolo-[3,4-c]-pyrrolen der Formel

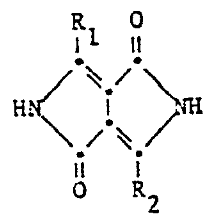

(I)

worin $R_1$ und $R_2$ unabhängig voneinander isocyclische oder heterocyclische Reste bedeuten, dadurch gekennzeichnet, dass man 1 Mol eines Bernsteinsäurediesters mit 2 Mol eines Nitrils der Formel

$$R_1\text{—}CN \text{ (II) oder } R_2\text{—}CN \qquad \text{(III)},$$

worin $R_1$ und $R_2$ die oben angegebene Bedeutung haben, oder mit 1 Mol eines Nitrils der Formel II und 1 Mol eines Nitrils der Formel III in einem organischen Lösungsmittel in Gegenwart einer starken Base bei erhöhter Temperatur umsetzt und aus dem entstandenen Reaktionsprodukt durch Hydrolyse die Verbindung der Formel I freisetzt.

2. Verfahren nach Anspruch 1, dadurch gekennzeichnet, dass man als Ausgangsstoff ein einheitliches Nitril der Formel II oder III verwendet.

3. Verfahren nach Anspruch 1, dadurch gekennzeichnet, dass man als Ausgangsstoffe Nitrile der Formel II und/oder III verwendet, worin $R_1$ und $R_2$ unsubstituiertes oder nicht-wasserlöslichmachende Substituenten aufweisendes Phenyl oder Naphthyl bedeuten.

4. Verfahren nach Anspruch 1, dadurch gekennzeichnet, dass man als Ausgangsstoffe Nitrile der Formel II und/oder III verwendet, worin $R_1$ und $R_2$ Reste aromatischer N-Heterocyclen bedeuten.

5. Verfahren nach Anspruch 1, dadurch gekennzeichnet, dass man als Ausgangsstoffe Nitrile der Formel II und/oder III verwendet, worin $R_1$ und $R_2$ o-, m- oder p-Pyridyl bedeuten.

6. Verfahren nach Anspruch 1, dadurch gekennzeichnet, dass man als Ausgangsstoffe Nitrile der Formel IV verwendet

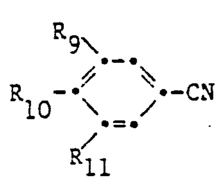

(IV)

worin $R_9$, $R_{10}$ und $R_{11}$ unabhängig voneinander Wasserstoff, Fluor, Chlor, Brom, Carbamoyl, Cyano, Trifluormethyl, $C_2$-$C_{13}$-Alkylcarbamoyl, $C_1$-$C_{12}$-Alkyl, $C_1$-$C_{12}$-Alkoxy, $C_1$-$C_{12}$-Alkylmercapto, $C_2$-$C_{13}$-Alkoxycarbonyl, $C_2$-$C_{13}$-Alkanoylamino, $C_1$-$C_{12}$-Monoalkylamino, $C_2$-$C_{24}$-Dialkylamino, unsubstituiertes oder durch Halogen, $C_1$-$C_{12}$-Alkyl oder $C_1$-$C_{12}$-Alkoxy substituiertes Phenoxy, Phenylmercapto, Phenoxycarbonyl, Phenylcarbamoyl oder Benzoylamino bedeuten, wobei mindestens einer der Substituenten $R_9$, $R_{10}$ und $R_{11}$ Wasserstoff bedeutet.

7. Verfahren nach Anspruch 1, dadurch gekennzeichnet, dass man als Ausgangsstoffe Nitrile der Formel V verwendet

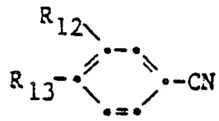

(V)

worin einer der Substituenten $R_{12}$ und $R_{13}$ Chlor, Brom, $C_1$-$C_4$-Alkyl, Cyano, $C_1$-$C_4$-Alkoxy, unsubstituiertes oder durch Chlor oder Methyl substituiertes Phenoxy, Carbamoyl, $C_2$-$C_5$-Alkylcarbamoyl, unsubstituiertes oder durch Chlor, Methyl oder Methoxy substituiertes Phenylcarbamoyl bedeutet, und der andere Wasserstoff ist.

8. Verfahren nach Anspruch 1, dadurch gekennzeichnet, dass man als Bernsteinsäurediester einen symmetrischen Bernsteinsäuredialkylester mit 1 bis 18 C-Atomen in jeder Alkylgruppe verwendet.

9. Verfahren nach Anspruch 1, dadurch gekennzeichnet, dass man als Bernsteinsäurediester einen symmetrischen Bernsteinsäuredialkylester verwendet, worin Alkyl sek.- oder tert.-Alkyl ist.

10. Verfahren nach Anspruch 1, dadurch gekennzeichnet, dass man als Lösungsmittel einen sekundären oder tertiären Alkohol verwendet.

11. Verfahren nach Anspruch 1, dadurch gekennzeichnet, dass man als starke Base ein Alkalialkoholat verwendet.

12. Verfahren nach Anspruch 1, dadurch gekennzeichnet, dass man die Reaktionspartner bei 60 bis 140 °C umsetzt.

13. Verfahren nach Anspruch 1, dadurch gekennzeichnet, dass man zur Hydrolyse eine organische Säure verwendet.

## Claims

1. A process for the preparation of a 1,4-diketopyrrolo-[3,4-c]-pyrrole of the formula

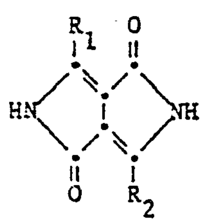

(I)

wherein each of $R_1$ and $R_2$ independently of the other is an isocyclic or heterocyclic radical, which process comprises reacting 1 mole of a disuccinate with 2 moles of a nitrile of the formula

$$R_1\text{—CN (II) or } R_2\text{—CN} \quad \text{(III)}$$

wherein $R_1$ and $R_2$ are as defined above, or with 1 mole of a nitrile of the formula (II) and 1 mole of the nitrile of the formula (III), in an organic solvent and in the presence of a strong base at elevated temperature, and obtaining the compound of formula I from the reaction product by hydrolysis.

2. A process according to claim 1, wherein the starting material is a homogeneous nitrile of the formula II or III.

3. A process according to claim 1, wherein the starting materials are nitriles of the formulae II and/or III, wherein $R_1$ and $R_2$ are unsubstituted phenyl or naphthyl or phenyl or naphthyl which carry non-watersolubilising substituents.

4. A process according to claim 1, wherein the starting materials are nitriles of the formulae II and/or III, wherein $R_1$ and $R_2$ are radicals of aromatic N-heterocyclic compounds.

5. A process according to claim 1, wherein the starting materials are nitriles of the formulae II and/or III, wherein $R_1$ and $R_2$ are o-, m- or p-pyridyl.

6. A process according to claim 1, wherein the starting materials are nitriles of the formula IV

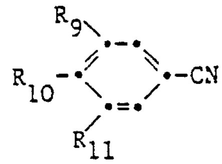

(IV)

wherein each of $R_9$, $R_{10}$ and $R_{11}$ independently of one another is hydrogen, fluorine, chlorine, bromine, carbamoyl, cyano, trifluoromethyl, $C_2$-$C_{13}$-alkylcarbamoyl, $C_1$-$C_{12}$-alkyl, $C_1$-$C_{12}$ alkoxy, $C_1$-$C_{12}$-alkylmercapto, $C_2$-$C_{13}$-alkoxycarbonyl, $C_2$-$C_{13}$-alkanoylamino, $C_1$-$C_{12}$-monoalkyl-amino, $C_2$-$C_{24}$-dialkylamino, or phenoxy, phenylmercapto, phenoxycarbonyl, phenylcarbamoyl or benzoylamino, each unsubstituted or substituted by halogen, $C_1$-$C_{12}$-alkyl or $C_1$-$C_{12}$-alkoxy, with the proviso that at least one of $R_9$, $R_{10}$ and $R_{11}$ is hydrogen.

7. A process according to claim 1, wherein the starting materials are nitriles of the formula V

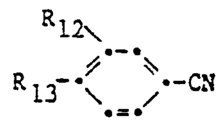

(V)

wherein one of $R_{12}$ and $R_{13}$ is chlorine, bromine, $C_1$-$C_4$-alkyl, cyano, $C_1$-$C_4$-alkoxy, or is phenoxy, carbamoyl or $C_2$-$C_5$-alkylcarbamoyl, each unsubstituted or substituted by chlorine or methyl, or is phenylcarbamoyl which is unsubstituted or substituted by chlorine, methyl or methoxy, and the other is hydrogen.

8. A process according to claim 1, wherein the disuccinate is a symmetrical dialkyl succinate containing 1 to 18 carbon atoms in each alkyl moiety.

9. A process according to Claim 1, wherein the disuccinate is a symmetrical dialkyl succinate, wherein alkyl is sec- or tert-alkyl.

10. A process according to claim 1, wherein the solvent is a secondary or tertiary alcohol.

11. A process according to claim 1, wherein the strong base is an alkali metal alcoholate.

12. A process according to claim 1, wherein the reactants are reacted in the temperature range from 60° to 140 °C.

13. A process according to claim 1, wherein an organic acid is used for the hydrolysis.

**Revendications**

1. Procédé pour la préparation de dioxo-1,4 pyrrolo-[3,4-c] pyrroles de formule

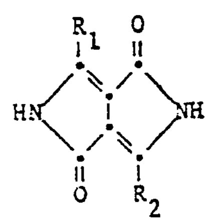

(I)

dans laquelle $R_1$ et $R_2$, indépendamment l'un de l'autre, sont chacun des radicaux isocycliques ou hétérocycliques, caractérisé en ce qu'on fait réagir 1 mole d'un diester de l'acide succinique sur 2 moles d'un nitrile de formule

$$R_1—CN(II) \text{ ou } R_2—CN \qquad (III)$$

où $R_1$ et $R_2$ ont les significations données ci-dessus, ou bien sur 1 mole d'un nitrile de formule II et 1 mole d'un nitrile de formule III dans un solvant organique, en présence d'une base forte et à haute température, et qu'on libère le composé de formule I, par hydrolyse, du produit de réaction qui s'est formé.

2. Procédé selon la revendication 1, caractérisé en ce qu'on utilise comme substance de départ un nitrile unique de formule II ou III.

3. Procédé selon la revendication 1, caractérisé en ce qu'on utilise comme substances de départ des nitriles de formules II et/ou III, dans lesquelles $R_1$ et $R_2$ sont chacun un radical phényle ou naphtyle non substitué ou présentant des substituants non solubilisants dans l'eau.

4. Procédé selon la revendication 1, caractérisé en ce qu'on utilise comme substances de départ des

nitriles de formule II et/ou III, dans lesquelles $R_1$ et $R_2$ sont chacun un hétérocycle azoté aromatique.

5. Procédé selon la revendication 1, caractérisé en ce qu'on utilise comme substances de départ des nitriles de formules II et/ou III, dans lesquelles $R_1$ et $R_2$ sont chacun le radical o-, m- ou p-pyridyle.

6. Procédé selon la revendication 1, caractérisé en ce qu'on utilise comme substances de départ des nitriles de formule IV

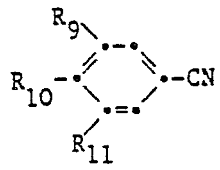

(IV)

dans laquelle $R_9$, $R_{10}$ et $R_{11}$, indépendamment les uns des autres, sont chacun l'hydrogène, le fluor, le chlore, le brome, les radicaux carbamoyle, cyano, trifluorométhyle, (alkyle en $C_2$-$C_{13}$)-carbamoyle, alkyle en $C_1$-$C_{12}$, alcoxy en $C_1$-$C_{12}$, alkylmercapto en $C_1$-$C_{12}$, (alcoxy en $C_2$-$C_{13}$) carbonyle, alcanoylamino en $C_2$-$C_{13}$, monoalkylamino en $C_1$-$C_{12}$, dialkylamino en $C_2$-$C_{24}$, phénoxy non substitué ou substitué par un halogène, un radical alkyle en $C_1$-$C_{12}$ ou alcoxy en $C_1$-$C_{12}$, un radical phénylmercapto, phénoxycarbonyle, phénylcarbamoyle ou benzoylamino, où au moins l'un des substituants $R_9$, $R_{10}$ et $R_{11}$ est l'hydrogène.

7. Procédé selon la revendication 1, caractérisé en ce qu'on utilise comme substances de départ des nitriles de formule V

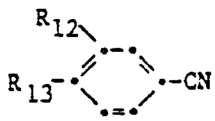

(V)

dans laquelle l'un des substituants $R_{12}$ et $R_{13}$ est le chlore, le brome, un radical alkyle en $C_1$-$C_4$, cyano, alcoxy en $C_1$-$C_4$, phénoxy non substitué ou substitué par le chlore ou le radical méthyle, carbamoyle, (alkyle en $C_2$-$C_5$)-carbamoyle, phénylcarbamoyle non substitué ou substitué par le chlore ou le radical méthyle ou méthoxy, et l'autre est l'hydrogène.

8. Procédé selon la revendication 1, caractérisé en ce qu'on utilise comme diester de l'acide succinique un ester dialkylique symétrique de l'acide succinique, possédant 1 à 8 atomes de carbone dans chaque groupe alkyle.

9. Procédé selon la revendication 1, caractérisé en ce qu'on utilise comme diester de l'acide succinique un ester dialkylique symétrique de l'acide succinique, où alkyle désigne un radical alkyle secondaire ou tertiaire.

10. Procédé selon la revendication 1, caractérisé en ce qu'on utilise comme solvant un alcool secondaire ou tertiaire.

11. Procédé selon la revendication 1, caractérisé en ce qu'on utilise comme base forte un alcoolate de métal alcalin.

12. Procédé selon la revendication 1, caractérisé en ce qu'on fait réagir à 60 à 140 °C les substances participant à la réaction.

13. Procédé selon la revendication 1, caractérisé en ce qu'on n'a besoin que d'un acide organique pour l'hydrolyse.